# EUROPEAN PATENT APPLICATION

(11) **EP 2 664 320 A1**
(43) Date of publication of application: **20.11.2013**
(21) Application number: 13180679.6
(22) Date of filing: 08.06.2004
(51) Int. Cl.: A61K 8/36, A61K 8/92, A61K 8/04, A61Q 19/00, A61Q 19/10, A61Q 9/02

(54) **Process for preparing a composition comprising unsaponifiable materials**

(30) Priority: 30.06.2003 US 611775
(62) Divisional of application: 04754840.9
(71) Applicant: International Flora Technologies, Ltd., Chandler, AZ 85225 (US)
(72) Inventor: Hill, John C., Mesa, AZ Arizona 85205 (US)
(74) Representative: Glas, Holger

(57) **Abstract**

The present invention is directed to a batch process using a steam kettle equipped with a propeller mixer. The produced composition comprises alkali salts of fatty acids, glycerine, and at least 6 weight percent unsaponifiable materials, and has a pH of greater than 7.

## Description

### Field of the Invention

The present invention relates to a novel composition of matter derived from natural materials or extracts of natural materials. In particular the invention relates to compositions derived from natural ingredients with relatively high levels of unsaponifiable materials (as defined below) and methods of using the same to activate gelling agents.

### Background of the Invention

Vegetable and animal fats are organic lipid materials that generally contain esters of long-chain fatty acids and glycerine. Under certain conditions these esters react with water (hydrolysis) to form an alcohol (glycerine) and fatty acids. (Hydrolysis is the splitting of a compound into components by the addition of water and an enzyme, acid or base.) The results of a hydrolysis reaction are known as "Hydrolysates". When heated in the presence of an alkali hydroxide the above mentioned esters yield soap (the alkali salt of the fatty acid) and glycerine; this particular hydrolysis process is called saponification. "Saponification" and "saponifying" are used herein in their normal manner to mean the hydrolysis reaction between a wax, oil or fat with an alkali metal or alkaline earth metal hydroxide to form the corresponding metallic salt soap. These fats and oils have a saponification value that is the number of milligrams of potassium hydroxide required for complete saponification of one gram of free organic acid and/or organic acid ester.

The post saponification products may either be hydrophilic (water soluble) or hydrophobic (water insoluble). Herein we will use the term "unsaponifiable" to mean those materials that, after the saponification reaction is completed, remain water insoluble. This is in full accord with the A.O.C.S. Official Method Ca 6b-53, which defines unsaponifiable materials as those substances frequently found as components of fats and oils, which cannot be saponified by the usual caustic treatment, but which are soluble in ordinary fats and oils. Included, but not limiting, in the group of unsaponifiable materials are higher aliphatic alcohols, sterols, pigments, mineral oils, and hydrocarbons. Unsaponifiable materials are generally non-volatile at 103°C. The weight percent of unsaponifiable material in a substance may be measured directly by measuring the weight percent of those materials defined as unsaponifiable.

The most well known vegetable and animal lipids have low levels, less than five percent (<5%), of unsaponifiable materials. This means that most of the products of the saponification reaction are water-soluble. Commonly used vegetable oils have levels of unsaponifiable materials generally below 1%. For example, saponification of soybean oil leaves 0.7 weight percent unsaponifiable materials, saponification of olive oil leaves 1.2 weight percent unsaponifiable materials, and saponification of peanut oil leaves 0.4 weight percent unsaponifiable materials. However, some commercial oils contain higher concentrations of unsaponifiable products, up to as much as 6.0 weight percent unsaponifiable materials. Examples include: crude rice bran oil, 4.2 % unsaponifiables, crude wheat germ oil, 6 % unsaponifiables, and shea butter, 9-13 % unsaponifiables. Materials with high levels of unsaponifiables, such as shea butter, are not a preferred starting material for the production of soap because of the high amount of unsaponifiable materials left after the saponification reaction.

In most cases, the hydrolysis products of a saponification process are used solely for a single purpose, which is as a hygienic skin-cleansing agent (soap). In the past, the basic ingredient of soap was animal fat (also known as lard or tallow) with wood ash based lye used in the saponification process. Ideally a bar of soap has a suitable hardness to maximize user cycles and has a certain amount of resistance to water reabsorption when not in use, while at the same time providing sufficient lather (acting as a foaming agent) to enhance the cleaning ability of the soap. Animal lipids as the active ingredient in the soap making process will generally meet these user demands to a greater or lesser degree. Current soap production continues to rely heavily on animal fats in their products to meet consumer demand and production requirements, although more and different types of synthetic materials are beginning to find use in soap compositions. The various synthetic compounds and mixtures of compounds have become very popular additions in modern soap making technology for their improvement to soap quality and user satisfaction. However, these synthetic based soaps are generally resistant to the natural breakdown processes (i.e. biodegradability) and are thus relatively persistent in the environment.

There are basically two distinct types of soap manufacturing processes. In a first method, oils and fats are boiled in an open kettle with caustic alkali solutions, bringing about saponification gradually until all of the fats and oils are completely saponified, followed by the removal of the glycerine. This process may either run in batch or in a continuous process.

In a second method, which is typically a continuous method (but may be run in batch form), fatty acids and alkali are brought together in proper portions for complete saponification in a mixing valve or other device which brings them in intimate contact. The progress of saponification depends on the temperature, time of contact and efficiency of mixing. Concentrated solutions produced by these methods are referred to as "neat" soaps, and possess a concentration of 60-65% soap, about 35% water and traces of salt and glycerine. It is from this product that consumer soaps in the form of bars, flakes, granules and powders are produced, by first drying the neat soap into pellets having a moisture content of about 12-16% followed by finishing steps, such as milling, plodding, amalgamating, and the like.

Consumer bar soaps today are manufactured from coconut oil and/or tallow or their fatty acids. Palm kernel oil is sometimes substituted for coconut oil for economic reasons, and soaps prepared with palm kernel oil are adjusted for performance characteristics similar to non-substituted tallow/coconut formulations. Palm oil is also often substituted for tallow.

A consideration in selecting materials for making soap is the proper ratio of saturated versus unsaturated, and long-versus-short-chain fatty acids that result in a soap having the desired qualities of stability, solubility, ease of lathering, hardness, cleaning ability, and the like. It has been determined that soaps prepared from fatty acid mixtures wherein a majority of the fatty acids in the mixtures has carbon chains less than twelve atoms irritate skin. Soaps prepared from saturated C₁₆ and C₁₈ fatty acids are typically too insoluble for consumer use. Thus, the preferred materials for soap production have fatty acid chains between twelve and eighteen carbon atoms in length.

Saponification of tallow produces a soap comprised of a mixture of fatty acids of C_{14:0}, C_{16:0}, C_{18:0}, and C_{18:1} (myristic, palmitic, stearic and oleic acids, respectively)and saponification of coconut oil produces a soap comprised of a mixture of fatty acids of C_{12:0} and C_{14:0} (lauric acid and myristic acid, respectively) and significant amounts of C_{8:0} and C_{10:0} fatty acids. Consumer soap preparations usually contain tallow/coconut (T/C) ratio ranges from approximately 90:10 to 75:25. Since lauric acid is found only in the coconut fraction of T/C mixtures, the most dramatic change observed in increasing the percent of the coconut fraction of T/C mixtures is the increase in the lauric acid. Increasing the coconut fraction in T/C fatty acid containing soaps generally improves the desirable foaming characteristics of such soaps. However, in soaps with T/C ratios of 50:50, the desirable skin mildness properties are reduced.

Typical fatty acid distribution (in weight percent) of the main soap making components is given below:

| Carbon Chain Length | Tallow | Palm | Coconut | Palm Kernel |
|---|---|---|---|---|
| 10:0 (capric) | 0.1 | 0.0 | 15.1 | 6.4 |
| 12:0 (lauric) | 0.1 | 0.3 | 48.0 | 46.7 |
| 14:0 (myristic) | 2.8 | 1.3 | 17.5 | 16.2 |
| 16:0 (palmitic) | 24.9 | 47.0 | 9.0 | 8.6 |
| 18:0 (stearic) | 20.4 | 4.5 | 9.0 | 8.6 |
| 18:1 (oleic) | 43.6 | 36.1 | 5.7 | 16.1 |
| 18:2 (linoleic) | 4.7 | 9.9 | 2.6 | 2.9 |
| 18:3 (linolenic) | 1.4 | 0.2 | 0.0 | 0.0 |
| 20:0 (arachidic) | 1.8 | 0.3 | 0.0 | 0.4 |

From the table it can be seen that the coconut and palm kernel fats (both known as the lauric fats) are particularly rich in the C₁₀₋₁₄ saturated fatty acids, particularly derivatives from lauric acid itself. Another fat that contains saturated, relatively short chain fatty acids similar to coconut oil is babassu oil. In contrast, tallow and palm oil *per se* are industrial sources of non-lauric fats, especially those containing C₁₆ and C₁₈ fatty acids.

In general the longer chain fatty acid alkali salts, particularly the less expensive C₁₆ and C₁₈ salts (as obtained from tallow and palm oils), provide structure in the finished soap bars and prevent or retard disintegration of the soap bar on exposure to water. The more expensive, shorter chain, lauric fat-derived, (i.e., lauric acid salts) and other soluble salts (typically as obtained from coconut and palm kernel oil) contribute to the lathering properties of the overall composition. A general problem in the formulation of bar soaps has been finding a balance between providing structure (generally obtained from the long chain component) and maintaining lathering properties (generally obtained from the more expensive short chain component) at a practical overall cost.

In addition to fatty acid salts, soap bars can contain free fatty acids. The addition of free fatty acids is known as 'superfatting'. Superfatting at a 5-10% free fatty acids level is known to give a copious, creamy lather. Other superfatting agents used include citric and other acids that function by promoting the formation of free fatty acids in the fat blend.

For the manufacture of the soap cakes, common additives can be added to the base soap in the normal quantities, referred to 100 parts by weight of base soap, such as overgreasing agents (1 to 3 wt.%), stabilizers (antioxidants, complexing agents) (0.05 to 0.5 wt.%), perfume (0.5 to 3 wt.%) and possibly dyes (0.05 to 0.3 wt.%) as well as skin protection agents such as sorbitol, glycerine or the like (1 to 5 wt.%).

The pharmaceutical and cosmetic industries have been using fat extracts of vegetable origin since earliest times. A number of years ago it became apparent in these industries that particularly valuable biological properties resulted from the use of vegetable fats or extracts of vegetable fats rich in unsaponifiable materials. Certain vegetable oils, for example avocado, and, in particular, shea butter, are known to be particularly rich in unsaponifiable materials and/or to contain these unsaponifiable materials.

A process for enriching unsaponifiables in oils, especially shea butter, for use in cosmetic and pharmaceutical compositions is described in U.S. Pat. No. 5,679,393, issued to Laur. This process concentrates the unsaponifiable fraction of fats and oils by the processes of crystallization and fractionation. This method is expensive and it does not liberate the alcohol moiety from the starting compounds (hydrolysis). Thus, the Laur process and methods for use of the products thereof never utilize hydrolysis to create alkali salts and liberate alcohols and other unsaponifiables.

Hydrolysates applied topically to animate and inanimate objects find use in numerous non-cleansing areas ranging from cosmetic preparations, pharmaceuticals, hydration formulations, insecticides, insect repellant, and the like. One of the areas of interest created by the varied uses of topically applied agents is maximizing the duration a topically applied active agent is present on the applied surface (substantivity). As a result of this intense interest, the search for ways to improve the duration of a fixed amount of topically applied cosmetics, pharmaceuticals, and bioactive agents has been of prime importance in all areas wherein topically applied cosmetics, pharmaceuticals, and bioactive agents are employed. An example of this interest may be found in the prior art relating to sunscreen compositions.

The use of sunscreen compositions is required by a large segment of society since only a small portion of those exposed to sunlight have the natural pigmentation which provides protection against the harmful effects of solar radiation. Because many people show erythema under even short exposures to sunlight, there is a need for sunscreen compositions that protect against erythema-causing radiation, i.e., ultraviolet radiation, so that longer exposure to the sunlight with less risk of sunburn is possible.

A variety of sunscreen compositions are known in the art. One tendency in formulating sunscreen compositions has been to prepare compositions that are water-resistant to the skin. One method is to chemically modify the ultraviolet absorber to increase its interaction with the skin by quaternizing imidazoles, as described in U.S. Pat. No. 3,506,758; another method is to copolymerize ultraviolet light absorbing monomers with other monomers to form water-resistant films, as described in U.S. Pat. Nos. 3,529,055 and 3,864,473; yet another method is to form polymeric films with water-insoluble polymers, as described in U.S. Pat. No. 3,784,488.

The use of the acid form of crosslinked ethylene-maleic anhydride copolymers to retain ultraviolet light absorbers is disclosed in U.S. Pat. No. 3,821,363. The use of water insoluble acrylate polymer having a solubility parameter of 6 to 10 in weak hydrogen bonding solvents is disclosed in U.S. Pat. No. 4,172,122. The use of water-insoluble, alcohol-soluble, film-forming poly-amide materials is disclosed in U.S. Pat. No. 3,895,104 solely for the purpose of providing improved substantivity..

The cosmetics and other applications of the prior art have not heretofore utilized the substantivity inherent in Hydrolysates of naturally derived materials containing high unsaponifiables or long chain esters (greater than 18 carbons in length) to enhance the intrinsic substantivity of topically applied agents with which they are incorporated. Previously, the purpose of employing polymers or polymeric materials in the compositions of the prior art has been directed towards improving the adherency, i.e., substantivity, of the topical material to the skin or have been employed solely as thickening agents. The improved substantivity, among other properties, achieved by employing the Hydrolysates according to the present invention has not heretofore been disclosed or appreciated in the prior art.

The increased substantivity of topically applied agents provides for more effective and economical use of such materials. In particular, the present invention provides improved compositions, including emollients, moisture retention agents, sunscreens, lipsticks, make up, insect repellants, insecticides, pesticides, herbicides, and the like, having at least an effective amount of a Hydrolysate including high levels of unsaponifiable materials.

Moreover, many of these compositions require the use of gelling or thickening agents. Typically these thickening agents are provided, prior to inclusion in the formulation, in an acidic aqueous solution. Gelling or thickening occurs when the pH of the solution (formulation) is neutralized to around a pH of 5.5 - 7.0, the gel viscosity being controlled by the pH.

The most commonly used neutralizers are: AMP (2-amino-2-methyl-1-propanol), AMPD (Aminomethyl propanediol, TIPA (Triisopropyl amine), DMS (Dimethyl Stearamine), DMHTA (Dimethyl hydrogenated tallow amine), TEA (Triethanolamine), NaOH (Sodium Hydroxide), KOH(Potassium Hydroxide), DEPA (Diethylpropylamine), DIPA (Diisopropanolamine), with the most common being TEA. However, health issues are being raised about many of the basic components. For example, TEA is being investigated as a potential cancer agent. The National Cancer Institute nominated TEA for study because of its prominent use in cosmetics and other consumer products, and its potential conversion into the carcinogen N-nitrosodienthanolamine. At this time, the conclusions of the dermal studies are undecided. However, dosed rats and mice had varying degrees of acanthosis (a thickening of the prickle cell layer of the epidermis) and inflammation, ulceration and epidermal erosion at the site of skin application.

Therefore, there is a need for compositions that can neutralize gelling solutions without known attendant health risks. Preferred compositions would also have increased substantivity and may even provide a degree of emolliency to the skin.

### Summary of the Invention

The hydrolysis of materials with high levels of unsaponifiable matter, such as extracts from plants, result in products with unique properties. It has been found that the application of hydrolysis products of materials, particularly naturally derived materials, with a high unsaponifiables fraction (e.g., at least 6% by total weight of the material) produces a Hydrolysate with properties that are significantly different from those products resulting from the conventional saponification of materials with less than 6% by weight of unsaponifiable.

The resulting products from the practice of the present invention are substantive, moisture retaining, prevent unwanted absorption of a carried active ingredient by the applied surface, exhibit a unique surfactant functionality, are not foaming agents with water and high pH suitable for acidic solution neutralization. Some unexpected uses for the resulting Hydrolysates have been found to be an acidic solution neutralizer that also acts as an emollient and/or an alternative natural carrying agent for topical application of cosmetics, pharmaceuticals, and bioactive agents, particularly to the skin of subjects, and provides a substantive support for the materials carried.

The novel features that are considered characteristic of the invention are set forth with particularity in the appended claims. The invention itself, however, both as to its structure and its operation together with the additional objects and advantages thereof will best be understood from the following description of the preferred embodiment of the present invention when read in conjunction with the accompanying drawings. Unless specifically noted, it is intended that the words and phrases in the specification and claims be given the ordinary and accustomed meaning to those of ordinary skill in the applicable art or arts. If any other meaning is intended, the specification will specifically state that a special meaning is being applied to a word or phrase. Likewise, the use of the words "function" or "means" in the Detailed Description of the Invention is not intended to indicate a desire to invoke the special provision of 35 U.S.C. §112, paragraph 6 to define the invention. To the contrary, if the provisions of 35 U.S.C. §112, paragraph 6, are sought to be invoked to define the invention(s), the claims will specifically state the phrases "means for" or "step for" and a function, without also reciting in such phrases any structure, material, or act in support of the function. Even when the claims recite a "means for" or "step for" performing a function, if they also recite any structure, material or acts in support of that means of step, then the intention is not to invoke the provisions of 35 U.S.C. §112, paragraph 6. Moreover, even if the provisions of 35 U.S.C. §112, paragraph 6, are invoked to define the inventions, it is intended that the inventions not be limited only to the specific structure, material or acts that are described in the preferred embodiments, but in addition, include any and all structures, materials or acts that perform the claimed function, along with any and all known or later-developed equivalent structures, materials or acts for performing the claimed function.

### Detailed Description of the Invention

The present invention is a composition of matter, and method for using the same, which is useful as a topically applied material with several useful inherent properties, such as a high pH and increased substantivity. Additionally, the composition is useful for carrying an effective amount of topically applied active materials. More specifically, the composition according the present invention provides a neutralizing agent for acidic gelling solutions and a carrying agent for the topical application of materials when superior "lasting" power or substantivity is required. Additionally, the present invention is useful because, among other things, it acts as both an emollient and unique emulsifier and demonstrates substantivity; it has the ability to "fix" many different types of "active" materials, from sunscreens to pharmaceutical preparations to any applied animate or inanimate surface.

For the purposes of this invention, the following definitions should be considered:
"High unsaponifiable materials" or "high unsaponifiable content" oils, waxes, fats, and the like, means compositions that comprises at least 6 % by weight of total organic materials that are unsaponifiable and at least 10 % by weight of organic materials that are saponifiable (it is possible that the percentage of unsaponifiables may even exceed 95% in some formulations). Therefore, the term includes compositions containing from 6-90% by weight of organics of unsaponifiable materials and 10-94% by weight of saponifiable materials. Examples of bio-based materials with high unsaponifiables are listed in the table below.

| **Material** | **% Unsaponifiables** |
|---|---|
| amaranth seed oil | 9% |
| anise seed oil | 7% |
| avocado seed oil | 57% |
| barley oil | 6% |
| briza oil | 78% |
| buck wheat oil | 7% |
| candelilla wax | 65-75% |
| carnuba wax | 50-55% |
| cassia occidentalis oil (wild coffee) | 7% |
| coffee bean oil | 8% |
| deoiled lecithin | 32% (in Theory) |
| dog fish oil | 16-18% |
| esparto wax | 42-49% |
| oils from fungi and other microorganisms | 6% or greater |
| guayule (plant material extract) | 8-12% |
| jojoba oil | 45% |
| jurinea oil | 40% |
| lanolin | 39% |
| laurel berry oil | 6% |
| olestra(TM)or olean(TM) | 33% (approximation) |
| olive oil concentrate (phytosqualene) | 35-75% |
| olive seed oil | greater than 6% |
| orange roughy oil | 40% |
| ouricury wax | 50-55% |
| quinoa seed oil | 6% |
| rye germ oil | 11% |
| shark liver oil | 60% |
| shea butter | 9-13% |
| sperm whale oil | 36% |
| sugar cane wax | 18-80% |
| sunflower wax | 25-45% |
| tall oil | 9-23% |
| tall oil distillate | 25-33% |
| Vegepure(TM) from wheat grains | 70-90% |
| wheat germ oil | 6% |

"Substantivity" means the tendency of a material to resist being easily removed or the persistence of a treatment on the skin. For example, some sunscreen lotions are substantive because they form a film on the skin that is relatively water-insoluble. This, then, means that substantive materials resist removal or transfer by physical contact, sweating or washing.

Compositions of matter comprising waxes, oils and/or fats (lipids) containing at least 6% by weight unsaponifiable ingredients and at least 10% by weight saponifiable ingredients are subjected to an alkaline hydrolysis reaction to produce a non-foaming, substantive composition with unique surfactant properties that may be used as an active ingredient or as a carrier for application of other active ingredients, e.g., as a carrier base for application of cosmetic, pharmaceutical or other active ingredients. Commercially available bio-based extracts that have high unsaponifiables include, but are not limited to, candelilla wax, carnuba wax, jojoba oil, lanolin, lecithin, and shea butter.

The lipid subjected to the process of the invention may be a raw product or it can also undergo various refining and/or modification steps beforehand. Examples of refining processes which may be mentioned are the conventional processes of chemical or physical refining or the more specialized processes for the refining of shea butter, which make it possible in particular to retain or concentrate the maximum amount of unsaponifiable materials, thereafter subjecting such treated materials to the process of the present invention.

The chemical refining which is preferentially used, being applied to the vegetable fats before they are subjected to the process according to the present invention, may be any conventional chemical refining process, in particular any process comprising the following steps:
Step 1: degumming involving insolubilization of the phosphatides with water, generally in the presence of acid, most frequently phosphoric acid, and separation by decantation or centrifugation (continuous process);
Step 2: neutralization of the free fatty acids in the oil by the addition of a sodium hydroxide solution and separation of the soaps formed (called soap stock), most frequently by centrifugation followed by several washes with water, steps 1 and 2 often being performed simultaneously in a continuous process;
Step 3: decolorization with activated bleaching clays at about 100°C under reduced vacuum, and filtration;
Step 4: deodorization operation necessary for removing the compounds responsible for the odors and flavors of an oil and for producing refined oil. This operation is carried out in an apparatus called a deodorizer, the procedure involving heating of the oil to a high temperature (180°-220°C.) under a vacuum of the order of 4 torr (i.e. about 532 Pa) and a massive injection of steam to strip away impurities.

An alternate physical refining method is understood as a variant of the chemical refining process explained above, the difference being that the neutralization step with sodium hydroxide is not performed and that the removal of the free fatty acids from the oil is effected during the deodorizing step. The refinement conditions selected during this physical refining method may require modification in order to retain the desired properties of the high unsaponifiables selected for use during the procedure for preparation of the present invention.

The extracts used as starting materials for the hydrolysis reaction according to the method of the present invention may be in their raw or refined states. The extracts may also be alkoxylated, polymerized, acetylated, oxidized, reduced, concentrated, hydrogenated, partial hydrogenated, interesterified, double bond modified, randomized, refined, or otherwise modified before the hydrolysis reaction. Since many lipids have low concentrations or fractions (for example 1% or less as discussed above) of unsaponifiables, the present invention encompasses the concentration of low fraction unsaponifiables into higher fractions, i.e., greater than 6%.

The products from the hydrolysis reaction of organic materials that produce unsaponifiables comprises a mixture of: a) polar hydrophilic salts (saponifiables); and b) non-polar, lipophilic materials (unsaponifiables), with the possibility of other materials also present, depending on the source, state and form of the initial reactant.

The composition of materials created by the method according to the present invention are produced by the reaction of aqueous alkali metal hydroxides, e.g., NaOH, LiOH, KOH (the preferred hydroxide), CaOH, MgOH, and the like, with organic lipid compositions, usually plant extracts, oils, fats, or waxes (of the extracts or derivatives of the extracts) where the organic compositions contain a high proportion of unsaponifiable materials (greater than 6%), and preferably as long chain esters.

Jojoba oil may be examined as an example case. Refined jojoba oil contains various proportions of long chain diunsaturated esters. Hydrolysates of refined jojoba oil are nearly a 55:45 mixture of polar hydrophilic long chain salts (alkali salts) and relatively non-polar lipophilic materials (fatty alcohols). The lipophilic fraction is the unsaponifiable materials according to the definition used in this document. The carbon chain lengths of both of these jojoba Hydrolysates include and vary from C₁₈ to C₂₄ and have ω-9 double bonds as part of each molecule. It has been found that the combination of saponifiable and unsaponifiable fractions of the Hydrolysates according to the present invention has properties that aid in the formulation of cosmetic, pharmaceutical, and other compositions.

The products that result from the hydrolysis of the lipids containing high percentages of unsaponifiable materials, as created during the practice of the present invention, whether used neat, blended, dissolved, dispersed, or emulsified with excipients, solvents, or carriers, can contain and impart useful properties to applied surfaces. These surfaces may be animate surfaces, particularly human skin, plant surfaces, and even the surfaces of inanimate objects, for example objects of wood, fiber, or plastic. The properties can include, but are not limited to, substantivity, emulsification, moisture retention, and the like.

One of the above-mentioned properties, substantivity, is particularly useful in the field of lipstick, shampoos, conditioners, hair sheens, repellants, attractants, cosmetics, pharmaceuticals, and sunscreens. The property of substantivity is especially beneficial to hair care products, such as "leave in" hair conditioners, where naturally derivatized materials that display substantivity are particularly commercially desirable. Substantivity is also particularly useful with sunscreen, sun block, or tanning formulations, as well as with insect repellants, such as tick, flea and fly repellants, and pesticides. Substantivity may also be beneficial when used on inanimate objects, such as with air fresheners, antibacterial, antimildew, and antifungal agents, flystrips, pesticides, insecticides, insect repellants, herbicides, and the like.

It is theorized that the inclusion of the high levels of unsaponifiable materials in the organic material enables the Hydrolysates according to the present invention to display their unique combination of properties. The precise nature of the unsaponifiable materials within the oils, waxes, fats or other natural extracts is not particularly important (except when a specific property is desired), and each of the variously available natural starting materials may differ significantly in their composition and types of unsaponifiables. For example, Jurinea extracts (e.g., the petroleum ether extracts of Jurinea) may comprise 40% by weight of pentacyclic triterpene alcohols together with their esters (myristate, palmitate, and acetate) as well as α-amyrin, β-amyrin, lupeol, and taraxasterol such as 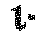 -taraxasterol (Lipids, K. L. Mikolajczak et al., 1967, Vol. 2, No. 2, pp. 127-132). Briza oil may contain 20% by weight of lipids that are semi-solid, the lipid comprising 49% unsaponifiable digalactosylglycerides, 29% unsaponifiable monogalactosylglycerides and small amounts of conventional saponifiable triglycerides. The predominant fatty acids in the above oils are palmitic acid, oleic acid and linoleic acid (Lipids, C. R. Smith, Jr. et al., March 1966, Vol. 1, No. 2, pp. 123-127).

It has been found that by providing aqueous alkali metal hydroxides, a basic solution, one having a high pH, may be produced. It is this high pH solution that is suitable for neutralizing (thickening) acidic gelling agents. It is understood by one of ordinary skill in the arts that the exact amount of high pH Hydrolysates, according to the present invention, needed to neutralize the acidic gelling agents depends upon the pH of the Hydrolysates and the amount, composition and pH of the gelling agents.

Preferred acidic gelling agents include, but are not limited to synthetic polymers, gums, hydrophilic colloids and their derivatives. Synthetic Polymers start with a raw material such as carbomers, acrylates copolymes, PVM/MA decadiene crosspolymers, acrylates/steareth-20 acrylates copolymer, and steareth-10 allyl ether/acrylates copolymers (or combinations thereof), Gums, hydrophilic colloids and their derivatives start with raw material such as cellulose or carbohydrate type derivatives. Examples of these types of raw materials include Gellan Gum, Xanthan Gum, Hydroxyethylcellulose, and Hydroxypropyl Guar. Gellan Gum by definition is a high molecular weight heteropolysaccharide gum produced by pure-culture fermentation of a carbohydrate with Pseudomonas elodea. Xanthan gum fits the same definition but is produced from Xanthomonas campestris.

The composition according to the present invention is preferably produced in a batch process using a large steam kettle equipped with a propeller mixer.

A measured quantity of potassium hydroxide pellets are added into the steam kettle with a measured quantity of distilled, deionized, or reverse osmosis purified water. The amount of potassium hydroxide employed to completely saponify the free organic acid and/or organic acid ester dan accordingly be calculated from the Saponification Value of the starting material and will, in theory, be the stoichiometric amount. In practice, however, it is preferred to employ slightly more than the stoichiometric amount of potassium hydroxide in order to ensure that the Hydrolysates that are formed contain unused alkali. The amount of potassium hydroxide employed can be considerably more than the stoichiometric amount, for example, as much as 150% of the stoichiometric amount or more may be used depending upon the desired result.

The potassium hydroxide pellets and water are stirred together with the propeller mixer until the potassium hydroxide pellets are dissolved. It is important to note, for safety purposes, that heat is generated during this step and the mixture is quite caustic. Individuals nearby should wear gloves, eye and face protection, and clothing protection to avoid burns, both thermal and chemical.

Next, a measured quantity of a refined or derivatized organic material containing a high proportion of unsaponifiables, such as jojoba oil, is gently added to the steam kettle, taking care not to splash the caustic solution contained therein.

The steam kettle is heated to 90-95°C and held at that temperature range under constant agitation for two hours. At this point, the resultant mixture should be pH tested. Continue heating the mixture under constant agitation at 90-95°C. Retest the solution periodically until the pH is stable at approximately 10.5. Once the pH is stable, withdraw a sample for analysis. This sample should be analyzed by such methods as chromatography or by another like or similar method, to show that the reaction has proceeded as desired.

The resultant Hydrolysate may then be diluted by adding a second measure quantity of water, or other diluent, to the steam kettle and stirred with the mixing propeller. Heat should be continuously applied, less than 80°C, until the mixture is homogeneous.

Once homogeneous, the Hydrolysate mixture is cooled to 60°C while continuing the mixing with the propeller. The Hydrolysate mixture may then be transferred to a holding container and allowed to cool to room temperature before sealing the holding container.

Below are described several example uses found for the Hydrolysates according to the present invention.

### Example 1 Hand Sanitizer with Extended Skin Moisturization

This formula produces a clear antibacterial gel delivering emolliency, and Vitamin E to leave hands moisturized, nourished and fresh. Enhanced moisturization is achieved through the use of the substantive composition according to the present invention. The fragrance note is extended with the use of the composition according to the present invention as well.

| **Phase** | **Trade Name** | **INCI Name** | ***Supplier*** | **% wt/wt** |
|---|---|---|---|---|
| A. | Deionized Water | Water | | Q.S. |
| | Carbopol ETD2020 | Acrylates/C10-30 Alkyl Acrylate Crosspolymer | Noveon | 0.3 |
| | Versene NA | Disodium EDTA | Dow | 0.01 |
| B. | Ethanol SDA-40 | Alcohol | Remet | 61.78 |
| C. | Ethanol SDA-40 | Alcohol | Remet | 0.22 |
| | Floraesters K-20W Jojoba | Hydrolyzed Jojoba Esters (and) Water (aqua) | Floratech | 1.00 |
| D. | Florasomes Jojoba MXS w/ 15% Fragrance | Jojoba Esters (and) Fragrance | Floratech | 0.62 |
| | Florasomes Jojoba SXS w/10% Vit. E | Jojoba Esters (and) Tocopherol Acetate | Floratech | 0.38 |
| | | TOTAL: | | 100.00 |

### Mixing Procedure:

1. At room temperature, add the Versene NA to water with propeller agitation. Add Carbopol ETD2020 slowly, and disperse with high-speed propeller agitation for one hour.
2. Add Phase B to Phase A and mix with moderate sweep agitation for 20 minutes. Let AB sit still for at least 1 hour to de-aerate.
3. Pre-mix Phase C and add to Phase AB using moderate sweep agitation until desired viscosity is achieved.
4. Wet Phase D using equal amount of ABC and add to ABC with moderate sweep agitation.
5. Mix using moderate sweep agitation for 15 minutes or until all of Phase D is distributed evenly throughout the batch.

A commercially available skin lotion was purchased and divided equally. Half was used as a control and half was used as a base into which 5% of a jojoba Hydrolysate was incorporated. The jojoba Hydrolysate was prepared according to the method disclosed in this invention. A baseline skin hydration reading was taken with the Nova Meter for each panelist in advance of any lotion application. The control and Hydrolysate containing lotions were applied to different areas of each panelist forearms. The Hydrolysate containing lotion was applied to the right forearm and the control lotion was applied to the left forearm. The Nova Meter was used to take skin Hydration readings of the forearm areas to which each participant had applied each lotion. Multiple skin hydration readings were taken and recorded at one-hour intervals after lotion application.

The experiment resulted in a dramatically extended time period for skin hydration for most all test subjects in the test areas where the Hydrolysate formulation was applied, compared to the test areas of the control formulation. In general, 6 to 10 hours after application, the Hydrolysate lotion formulation demonstrated a 20% to 54% improvement in moisture content over baseline areas. The Hydrolysate formulation showed a 10% to 47% improvement in moisture content over skin treated with the control formulation.

### Example 2 - Skin Conditioning Shave Gel

This unique shave gel does not rely on foam or lather to provide a clean, close shave. Instead, the shave gel lubricates the skin and beard in a way that only botanicals can. Razor friction is reduced, the closeness of the shave is enhanced, and irritation is virtually eliminated when this gel is used. This substantive gel provides enhanced skin moisturization for hours after the shave.

| **Phase** | **Trade Name** | **INCI Name** | **Supplier** | **%wt./wt.** |
|---|---|---|---|---|
| A. | Deionized Water | Water | | 62.74 |
| | Carbopol ETD 2020 | Acrylates/C10-30 Alkyl Acrylate Crosspolymer | Noveon | 0.60 |
| | Versene NA | Disodium EDTA | Dow | 0.01 |
| B. | Sorbitol | Sorbitol | SPI Polyols | 10.00 |
| | Florasolvs^{®} PEG-120 Jojoba | Jojoba Wax PEG-120 Esters | Floratech | 5.00 |
| | Glycerin | Glycerin | Spectrum | 5.00 |
| C. | Butylene Glycol | Butylene Glycol | Ashland | 1.00 |
| | Preservative | ----- | | Q.S. |
| | Fragrance for Skin Care (13765H) | Fragrance | Shaw Mudge & Co. | Q.S. |
| D. | SD Alcohol 40 | SD Alcohol 40 | Remet | 10.00 |
| | Floraesters^{®} K-20W Jojoba | Hydrolyzed Jojoba Esters (and) Water (aqua) | Floratech | 5.00 |
| | Color (optional) | - - - - - | | Q.S. |
| | | Total: | | 100.0 |

### Mixing Procedure:

1. Add Versene NA to DI water with propeller mixing. Slowly sift the Carbopol into the DI water and allow enough mix time for complete hydration to take place.
2. Mix components of Phase B together at 60°C. Continue to mix Phase A and raise that temperature to 60°C. Add Phase B to Phase A and mix for 20 minutes at 60°C. Lower temperature to 40°C
3. Premix the methyl and propylparaben in the butylene glycol, and add to Phase AB. Add fragrance if required.
4. Premix the Floraesters K-20W in ethanol and add to Phase ABC. Add color if required. The incorporation of the Hydrolysates according to the present invention into typically drying shaving gel formations shows improved moisture retention properties compared to formulations not containing the Hydrolysates. Additionally, the incorporation of the Hydrolysates according to the present invention adds perceptive degrees of lubrication to the shaving process.

### Example 3 - Skin Conditioning Shower Gel

A clear, cleansing shower gel that leaves the skin feeling silky smooth and moisturized after every shower. Florapearls and Florabeads add to the visual effect of the product and provide a scrubbing sensation during the shower experience.

| ***Phase*** | **Trade Name** | **INCI Name** | **Supplier** | **%wt./wt.** |
|---|---|---|---|---|
| **A.** | Deionized Water | Water | | Q.S |
| | Versene NA | Disodium EDTA | Dow | 0.1 |
| | Carbopol Aqua SF-1 Polymer | Acrylates Copolymer | Noveon | 10.0 |
| | Florasolvs PEG-16 Macadamia | PEG-16 Macadamia Glycerides | Floratech | 3.0 |
| | Sulfochem ES-2 | Sodium Laureth Sulfate | Chemron | 25.0 |
| **B.** | Sulfochem AEG | Ammonium Lauryl Sulfate (and) ALES (and) CAPB (and) | Chemron | 4.5 |
| | | Cocamide DEA (and) Lauramide DEA | | |
| | Solubilisant LRI | PPG-26-Buteth-26 (and) PEG-40 Hydrogenated Castor Oil | LCW | 1.0 |
| | Floraesters K-20W Jojoba | Hydrolyzed Jojoba Esters | Floratech | 7.5 |
| | Standamid LD | Lauramide DEA | Cognis | 3.7 |
| | Propylene Glycol | Propylene Glycol | Ashland | 5.0 |
| **C.** | Lexaine C | Cocamidopropyl Betaine | Inolex | 5.0 |
| **D.** | Tween 20 | Polysorbate 20 | Uniqema | 0.8 |
| | Fragrance | Fragrance | Intercontinental Fragrances | Q.S |
| **E.** | Preservative | - - - - - | | Q.S. |
| | | 18 | | |
| | Florapearls Jojoba STD | Jojoba Esters | Floratech | Q.S. |
| | Florabeads Jojoba 28/60 | Jojoba Esters | Floratech | Q.S. |
| | | TOTAL: | | 100.0 |

### Mixing Procedure:

1. Put Versene EDTA into water and mix until dissolved. Add Aqua SF-1 polymer with medium propeller agitation. Mix in PEG-16 Macadamia and sodium laureth sulfate and allow 30 minutes mix time to insure complete mixing.
2. Mix Phase B together at room temperature allowing sufficient time for the K-20W to completely dissolve in the liquid. Add Phase B to Phase A with slow hand mixing to prevent excess air bubbles from becoming trapped in the mix.
3. Add Phase C to Phase AB with gentle hand mixing.
4. Mix the fragrance into the Polysorbate 20 and add Phase D to Phase ABC with gentle hand mixing.
5. Add Phase E to Phase ABCD with complete hand mixing to insure total distribution of the Florapearls and Florabeads in the base mix.

The preferred embodiment(s) of the invention is described above in the Detailed Description of the Invention. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventor that the words and phrases in the specification and claims be given the ordinary and accustomed meanings to those of ordinary skill in the applicable art(s). The foregoing description of a preferred embodiment and best mode of the invention known to the applicant at the time of filing the application has been presented and is intended for the purposes of illustration and description. It is not intended to be exhaustive or to limit the invention to the precise form disclosed, and many modifications and variations are possible in the light of the above teachings. The embodiment was chosen and described in order to best explain the principles of the invention and its practical application and to enable others skilled in the art to best utilize the invention in various embodiments and with various modifications as are suited to the particular use contemplated.

In view of the above, some aspects of the invention relate to:
1. A composition comprising alkali salts of fatty acids, glycerine, and at least 6 weight percent unsaponifiable materials, wherein the composition has a pH greater than 7 and the substantivity is greater than a like composition omitting the at least 6 weight percent unsaponifiable materials.
2. The composition of aspect 1 wherein said fatty acids are obtained from the group consisting of amaranth seed oil, anise seed oil, avocado seed oil, barley oil, briza oil, buck wheat oil, candelilla wax, carnuba wax, cassia occidentalis oil, coffee bean oil, deoiled lecithin, dog fish oil, esparto wax, oils from fungi and other microorganisms, guayule plant extract, jojoba oil, jurinea oil, lanolin, laurel berry oil, olestra (olean), olive oil concentrate (phytosqualene), olive seed oil, orange roughy oil, ouricury wax, quinoa seed oil, rye germ oil, shark liver oil, shea butter, sperm whale oil, sugar cane wax, sunflower wax, tall oil, tall oil distillate, Vegepure from wheat grains, and wheat germ oil.
3. The composition of aspect 1 comprising at least 20% by weight of unsaponifiables.
4. The composition of aspect 2 comprising at least 20% by weight of unsaponifiables.
5. A method of providing substantive benefits to an animal subject comprising adding a suitable amount of the composition of aspect 1 to an acidic gelling agent in order to neutralize the acid gelling agent followed by applying the resultant neutralized gelling agent to the hair, skin, scales, or feathers of an animal subject.
6. A method of providing substantive benefits to an animal subject comprising adding a suitable amount of the composition of aspect 2 to an acidic gelling agent in order to neutralize the acid gelling agent followed by applying the resultant neutralized gelling agent to the hair, skin, scales, or feathers of an animal subject.
7. A method of providing substantive benefits to a botanical subject comprising adding a suitable amount of the composition of aspect 1 to an acidic gelling agent in order to neutralize the acid gelling agent followed by applying the resultant neutralized gelling agent to the hair, skin, scales, or feathers of an animal subject.
8. A method of providing substantive benefits to a botanical subject comprising adding a suitable amount of the composition of aspect 2 to an acidic gelling agent in order to neutralize the acid gelling agent followed by applying the resultant neutralized gelling agent to the hair, skin, scales, or feathers of an animal subject.
9. A method of providing substantive benefits to an inanimate subject comprising adding a suitable amount of the composition of aspect 1 to an acidic gelling agent in order to neutralize the acid gelling agent followed by applying the resultant neutralized gelling agent to the hair, skin, scales, or feathers of an animal subject.
10. A method of providing substantive benefits to an inanimate subject comprising adding a suitable amount of the composition of aspect 2 to an acidic gelling agent in order to neutralize the acid gelling agent followed by applying the resultant neutralized gelling agent to the hair, skin, scales, or feathers of an animal subject.
11. A composition for topical application comprising alkali salts of fatty acids, glycerine, and at least 6 weight percent unsaponifiable materials, wherein the composition has a pH greater than 7 and the substantivity is greater than a like composition omitting the at least 6 weight percent unsaponifiable materials, said unsaponifiable materials being at least 18 carbons in length.
12. The composition of aspect 11 wherein said alkali salts of fatty acids, glycerine, and at least 6 weight percent unsaponifiable materials further are obtained from extracts selected from the group consisting of amaranth seed oil, anise seed oil, avocado seed oil, barley oil, briza oil, buck wheat oil, candelilla wax, carnuba wax, cassia occidentalis oil, coffee bean oil, deoiled lecithin, dog fish oil, esparto wax, oils from fungi and other microorganisms, guayule plant extract, jojoba oil, jurinea oil, lanolin, laurel berry oil, olestra (olean), olive oil concentrate (phytosqualene), olive seed oil, orange roughy oil, ouricury wax, quinoa seed oil, rye germ oil, shark liver oil, shea butter, sperm whale oil, sugar cane wax, sunflower wax, tall oil, tall oil distillate, Vegepure from wheat grains, and wheat germ oil.
13. The composition of aspect 11 comprising at least 20% by weight of unsaponifiables.
14. The composition of aspect 12 comprising at least 20% by weight of unsaponifiables.
15. A substantive composition comprising the composition of aspect 11 in combination with at least one ingredient selected from the group consisting of emollients, conditioners, pigments, dyes, pharmaceuticals, ultraviolet radiation absorbers, physical radiation blocks, insect repellants, animal repellants, insecticides, pesticides, herbicides, animal attractants, fragrances, and hormones.
16. A substantive composition comprising the composition of aspect 12 in combination with at least one ingredient selected from the group consisting of emollients, conditioners, pigments, dyes, pharmaceuticals, ultraviolet radiation absorbers, physical radiation blocks, insect repellants, animal repellants, insecticides, pesticides, herbicides, animal attractants, fragrances, and hormones.
17. A method of providing substantive benefits to an animal subject comprising adding a suitable amount of the composition of aspect 11 to an acidic gelling agent in order to neutralize the acid gelling agent followed by applying the resultant neutralized gelling agent to the hair, skin, scales, or feathers of an animal subject.
18. A method of providing substantive benefits to an animal subject comprising adding a suitable amount of the composition of aspect 12 to an acidic gelling agent in order to neutralize the acid gelling agent followed by applying the resultant neutralized gelling agent to the hair, skin, scales, or feathers of an animal subject.
19. A method of providing substantive benefits to a botanical subject comprising adding a suitable amount of the composition of aspect 11 to an acidic gelling agent in order to neutralize the acid gelling agent followed by applying the resultant neutralized gelling agent to the hair, skin, scales, or feathers of an animal subject.
20. A method of providing substantive benefits to a botanical subject comprising adding a suitable amount of the composition of aspect 12 to an acidic gelling agent in order to neutralize the acid gelling agent followed by applying the resultant neutralized gelling agent to the hair, skin, scales, or feathers of an animal subject.
21. A method of providing substantive benefits to an inanimate subject comprising adding a suitable amount of the composition of aspect 11 to an acidic gelling agent in order to neutralize the acid gelling agent followed by applying the resultant neutralized gelling agent to the hair, skin, scales, or feathers of an animal subject.
22. A method of providing substantive benefits to an inanimate subject comprising adding a suitable amount of the composition of aspect 12 to an acidic gelling agent in order to neutralize the acid gelling agent followed by applying the resultant neutralized gelling agent to the hair, skin, scales, or feathers of an animal subject.

## Claims

1. A method for producing a composition in a batch process using a steam kettle equipped with a propeller mixer, comprising the steps of:
i) adding a measured quantity of potassium hydroxide pellets into the steam kettle with a measured quantity of distilled, deionized, or reverse osmosis purified water,
ii) stirring the potassium hydroxide pellets and water until the potassium hydroxide pellets are dissolved,
iii) adding a measured quantity of a refined or derivatized organic material containing at least 6 wt.-% of unsaponifiables to the steam kettle,
iv) heating the steam kettle to 90-95°C and holding that temperature range under constant agitation until the pH is stable at 10.5,
wherein the amount of potassium hydroxide is 150% of the stoichiometric amount or more, which is employed to completely saponify the free organic acid and/or organic ester contained in the organic material.

2. The method of claim 1, further comprising the step of diluting the obtained composition by adding a second measure quantity of water to the steam kettle and stirring the composition.

3. The method of claims 1 or 2, further comprising the step of cooling the obtained composition to 60°C while stirring the composition, transferring the obtained composition to a holding container, and allowing the obtained composition to cool to room temperature before sealing the holding container.

4. The method of any one of the preceding claims, wherein the organic material is selected from the group consisting of amaranth seed oil, anise seed oil, avocado seed oil, barley oil, briza oil, buck wheat oil, candelilla wax, carnuba wax, cassia occidentalis oil, coffee bean oil, deoiled lecithin, dog fish oil, esparto wax, oils from fungi and other microorganisms, guayule plant extract, jojoba oil, jurinea oil, lanolin, laurel berry oil, olestra, olive oil concentrate, olive seed oil, orange roughy oil, ouricury wax, quinoa seed oil, rye germ oil, shark liver oil, shea butter, sperm whale oil, sugar cane wax, sunflower wax, tall oil, tall oil distillate, and wheat germ oil.

5. The method of any one of the preceding claims, wherein the produced composition comprises alkali salts of fatty acids, glycerine, and at least 6 weight percent unsaponifiable materials, and wherein the composition has a pH of greater than 7.
